(19) Europäisches Patentamt
European Patent Office
Office européen des brevets

(11) Publication number: **0 098 396**
**B1**

(12) # EUROPEAN PATENT SPECIFICATION

(45) Date of publication of patent specification: **07.01.88**

(51) Int. Cl.[4]: **C 07 C 126/02**

(21) Application number: **83105592.6**

(22) Date of filing: **07.06.83**

(54) **Process for the displacement from the liquid to the gaseous phase of the excess of NH3 contained in aqueous solutions of urea.**

(30) Priority: **08.06.82 IT 2177482**

(43) Date of publication of application:
**18.01.84 Bulletin 84/03**

(45) Publication of the grant of the patent:
**07.01.88 Bulletin 88/01**

(84) Designated Contracting States:
**BE DE FR GB IT NL**

(56) References cited:
**GB-A-2 089 786**
**US-A-4 013 718**
**US-A-4 208 347**

(73) Proprietor: **Montedison S.p.A.**
**31, Foro Buonaparte**
**I-20121 Milan (IT)**

(72) Inventor: **Pagani, Giorgio**
**26/6 V. le Faenza**
**Milano (IT)**

(74) Representative: **Barz, Peter, Dr. et al**
**Patentanwälte Dr. V. Schmied-Kowarzik Dipl.-Ing. G. Dannenberg Dr. P. Weinhold Dr. D. Gudel Dipl.-Ing. S. Schubert Dr. P. Barz**
**Siegfriedstrasse 8**
**D-8000 München 40 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 098 396 B1

## Description

The invention is an improvement of the process disclosed in U.S. patent 4 208 347 and of other analogous processes. Said U.S. patent concerns a synthesis of urea by reacting $NH_3$ and $CO_2$ in the presence of an excess amount of $NH_3$, with intermediate formation of ammonium carbamate, and provides for the displacement of the non-reacted reactants from the aqueous urea solution leaving the synthesis reactor by means of two subsequent strippings, isobaric with the synthesis; in the first step the synthesis product is heated and optionally brought into contact with an $NH_3$ stream and in the second step the same product is heated and brought into contact with a $CO_2$ stream. In the first step a major proportion of carbamate and part of the free (non-reacted) $NH_3$ are removed by supplying heat and by injecting an $NH_3$ stream as stripping agent; it is thus possible to obtain an urea solution containing a relatively low amount of $CO_2$, but a huge amount of residual $NH_3$, bound just to the operating conditions, which provide for an excess of $NH_3$. In the second step nearly all the residual $NH_3$ still present is removed by a second supply of heat and by using, as a stripping agent, the $CO_2$ feed required by the synthesis, while no appreciable change does occur in the carbamate percentage, already brought to a sufficiently low level in the first stripping step, and this is proved by the experimental tests reported in said U.S. patent. In said first isobaric stripper, heat is supplied by means of condensing steam but also by exploiting the heat content of the solution, which undergoes therefore a cooling U.S. patent 4 208 347, which has marked a basilar turning-point in the technology of the urea synthesis, is valuable for having shown, first in the world, that the elimination of the $NH_3$ excess, independently from the destiny of the last traces of carbamate, is the primary and natural purpose of said second stripping ($CO_2$ stripping), subsequent to the preceding (first) stripping, all this being contrary to the teachings of several other patents, filed also later than said U.S. patent, which strongly recommend to consider, as main object and purpose of the second stripping ($CO_2$ stripping), the other (actually minor) concomitant effect of the $CO_2$ treatment, namely the decomposition of the still present carbamate.

An object of the present invention is to improve the manufacturing loop described in said U.S. patent 4 208 347, frequently reported as "I.D.R." loop or "I.D.R." cycle (it is however possible to employ successfully the invention for other analogous loops).

In its most general form, the invention concerns a process for the removal, from the liquid to the gaseous phase, at a pressure of from 78.4 to 245.2 bar (80 to 250 Kg/cm$^2$), of the excess $NH_3$ contained in aqueous urea solutions having a temperature of from 150 to 230°C and coming from a preceding stripping (isobaric with the synthesis of urea from $NH_3$ and $CO_2$), said preceding stripping being performed in the presence of $NH_3$, with or without an injection of fresh $NH_3$ from the exterior, wherein said solutions contain also $CO_2$ (as ammonium carbamate), the $CO_2$ amount being equal to or lower than 25%, preferably 22% by weight, with respect to urea, and wherein said removal is performed by means of stripping in countercurrent with a $CO_2$ stream in a falling-film heat-exchanger containing a vertical tube bundle (hereinafter $CO_2$ stripper) which is also isobaric with said urea synthesis and is heated by supplying steam, the amount of ammonium carbamate in the solution leaving the bottom of the $CO_2$ stripper being substantially equal to the amount of ammonium carbamate in the solution entering the $CO_2$ stripper, characterized in that:

a) the global $NH_3$:$CO_2$ ratio in the solution fed to the $CO_2$ stripper is from 2 to 8, preferably from 3 to 6 and even better from 4 to 5.5 by weight; and

b) heat is supplied by means of steam substantially only to the uppermost portion of the tubes of the $CO_2$ stripper, the ratio between the surface of the lowermost portion and the surface of the uppermost portion of the tubes being at least 1:1 and the ratio between the residence time of the solution to be stripped in said lowermost portion and the residence time in said uppermost portion being at least 1:1, said residence time in the lowermost portion of the tubes being at least 3 seconds and said residence time in the uppermost portion being at the most 3 seconds.

Said solutions containing less than 25% by weight of carbamate come from at least one preceding isobaric stripping, for instance an $NH_3$ isobaric stripping; the invention however can be used not only in those double stripping loops in which the strippings are isobaric with the synthesis, but also in those manufacturing loops in which one or both of the strippings are at a lower or otherwise different pressure, always with respect to the synthesis. In any case, the instant invention is always valuable when the main purpose is to transfer into the gaseous phase the free $NH_3$ contained in an aqueous urea solution, at a rather high pressure, while using, as stripping agent, part or all of the carbon dioxide required by the urea synthesis.

The teaching of Canadian patent 1 113 961 is to heat strongly the uppermost portion of the tubes of a $CO_2$ stripper and to keep the residence time of the process fluid, in said heated uppermost portion, at a much higher level (5 s) with respect to the residence time (from 1 to 2 s) in the underlying portion, cooled or adiabatic, in order to "decompose" as much residual carbamate as possible.

According to the present invention, on the contrary, the residence time in the uppermost and heated portion must be lower (from 1 to 3 s) with respect to the underlying adiabatic portion (from 3 to 5 s) and the temperature drops in said underlying portion (partially because of the $CO_2$ addition) down to a level between 150 and 210, preferably 160 and 190 and even better 160 and 175°C, the temperature of the $CO_2$, injected as a stripping agent, being preferably from 80 to 140°C.

All this can be carried out, by heating only a small portion of the length of the tubes, namely a portion equal to or lower than 1/2 (preferably 1/3) and even better from 1/5 to 1/3 of the whole length, the underlying adiabatic portion being obviously the complement to 1. The invention allows excellent and unexpected results as to thermal balance, steam requirement and absence of corrosion; it allows further to stop completely and safely the degrading hydration of urea to ammonium carbamate, which can occur according to the known equation:

$$NH_2—CO—NH_2+H_2O \rightarrow NH_2—COONH_4.$$

Best results are reached when the ratio between the exchanging surface of the $CO_2$ stripper ($S_{CO2}$) and the exchanging surface ($S_{NH3}$) of the preceding stripper is given by the expression: $A=S_{CO_2}/S_{NH_3}>1$.

A preferred embodiment of the invention is a process for the removal, from the liquid to the gaseous phase, at pressures of from 127.4 to 215.8 bar (130 to 220 kg/cm$^2$), of the excess $NH_3$ contained in aqueous urea solutions having a temperature from 190 to 220°C and coming from a preceding stripping (isobaric with the synthesis of urea from $NH_3$ and $CO_2$), said preceding stripping being performed in the presence of $NH_3$, wherein said solutions contain also $CO_2$, the $CO_2$ amount being equal to or lower than 22% by weight with respect to urea, and wherein said removal is performed within a $CO_2$ stripper which is also isobaric with said urea synthesis and is heated by supplying steam, the amount of the carbamate in the solution leaving the bottom of the $CO_2$ stripper being substantially equal to the amount of carbamate in the solution entering the $CO_2$ stripper, characterized in that:

a) the $NH_3$:$CO_2$ ratio in the solution fed to the $CO_2$ stripper is from 3 to 6 (preferably from 4 to 5.5) by weight; and

b) heat is supplied by means of steam only to the uppermost portion of the tubes of the $CO_2$ stripper, the ratio between the surface of the lowermost portion and the surface of the uppermost portion of the tubes being at least 2:1 (preferably from 2:1 to 4:1) and the ratio between the residence time of the solution to be stripped in said lowermost portion and the residence time in said uppermost portion being at least 2:1 (preferably from 2:1 to 4:1), said residence time in the lowermost portion of the tubes being at least 3.5 (preferably from 3.5 to 5) s and said residence time in the uppermost portion being at the most 2.5 (preferably from 1.5 to 2.5) s.

The improved process of the present invention is still further illustrated by the accompanying figure 1, representing a simplified flow-sheet of the process itself. According to figure 1, urea is synthesized in a reactor subdivided into two overlying sections, fitted with sieve trays. The liquid product flows over by gravity into the underlying section through downcomer (18) and then it passes through pipe (4) in an isobaric falling-film exchanger ($NH_3$ stripper) containing a tube bundle, where the residual non-reacted carbamate is almost totally decomposed by means of an $NH_3$ stripping stream (21); the decomposition gases ($NH_3$ and $CO_2$) and part of the excess $NH_3$ flow back to the reactor (line 3). The solution (6) leaving the bottom of the $NH_3$ stripper is transferred to an isobaric $CO_2$ stripper, similar to the former ($NH_3$) stripper but having a greater number of exchanger pipes; in the $CO_2$ stripper part or the whole of the $CO_2$ amount required by the synthesis is injected, usually at 80—140°C, to the bottom of the stripper and removes nearly all the last traces of $NH_3$. The solution (7) leaving the stripper's bottom is conveyed to further conventional steps of the manufacturing process, while the vapor stream (9) leaves the head of the $CO_2$ stripper and is conveyed to a condenser, in admixture with the gases (13) coming from the reactor's head and with an aqueous solution (8) coming from further conventional low-pressure process steps. A stream of residual gases (14) leaves the condenser, while a carbamate solution (5) is recycled to the reactor merely by means of gravity and without needing any large and complicated recycle pump.

The $NH_3$ needed is pre-heated and fed to the reactor (line 1); in some cases a predetermined proportion of the $NH_3$ feed (19) is super-heated and fed to the lowermost section of the reactor. It is thus possible to obtain a final urea solution (7) rich in urea and having satisfactorily low biuret percentages.

The following examples further illustrate the invention, without however limiting the scope thereof.

Example 1

Following figure 1, urea is synthesized in a reactor subdivided into two each other overlying sections, fitted with sieve trays. The liquid product flows over by gravity into the underlying section through downcomer (18) and then it passes through pipe (4) into an isobaric falling-film exchanger containing a tube bundle, where the non-reacted carbamate is almost totally decomposed by means of an $NH_3$ stripping stream; the decomposition gases ($NH_3$ and $CO_2$) and part of the excess $NH_3$ flow back to the reactor (line 3). The solution (6) leaving the bottom of the $NH_3$ stripper contains 22% by weight of $CO_2$, with respect to urea (the 22% figure refers to $CO_2$, also if actually $CO_2$ is present as ammonium carbamate), and is then transferred to an isobaric $CO_2$ stripper which is similar to the $NH_3$ stripper but has a greater number of exchanger pipes; in the $CO_2$ stripper the whole $CO_2$ amount required for the synthesis is injected to the bottom of the stripper and removes nearly all of the last traces of $NH_3$. The solution (7) leaving the stripper's bottom is conveyed to further conventional steps of the manufacturing process. The vapor stream (9) leaves the head of the $CO_2$ stripper and is conveyed to a condenser, in admixture with the gases (13) coming from the reactor's head and with an aqueous solution (8) coming from further conventional low-pressure process steps; a stream of residual gases (14) leaves the condenser, while a carbamate

solution (5) is recycled to the reactor merely by means of gravity and without needing any recycle pump. The $NH_3$ feed is pre-heated and fed to the reactor (line 1); it is thus possible to obtain a final urea solution (7) rich in urea and having a satisfactorily low biuret percentage. 34513 kg/h of an aqueous urea solution (6), the composition of which is reported in Table 1, leave at 205°C and 196.1 bar (200 kg/cm$^2$), the bottom of the first isobaric stripper ($NH_3$ stripper), heated by saturated steam (10) at 24.5 bar (25 kg/cm$^2$); 9694 kg/h of $CO_2$ (line 2) enter at 130°C and 196.1 bar (200 kg/cm$^2$), the bottom of the second isobaric stripper ($CO_2$ stripper), heated too by steam at 24.5 bar (25 kg/cm$^2$) only in the uppermost section of the tube-bundle; 25580 kg/h of a final aqueous solution (7), the composition of which is in Table 1, leave, at 172°C and 196.1 bar (200 kg/cm$^2$), the bottom of the same $CO_2$ stripper. Further, 18627 kg/h of the fluid (9), the composition of which is in table 1, leave, at 200°C, the head of the same $CO_2$ stripper; the ratio between the amount of carbamate (CBRU) leaving the $CO_2$ stripper and the amount of carbamate (CBRE) entering the same $CO_2$ stripper is: CBRU:CBRE×100=2930:2698×100=108,6%. The small amount of steam (11) required for the heating of the $CO_2$ stripper is such as to comply with the thermal balanace. The low-pressure steam (12) generated in the condenser is at about 5.9 bar (6 kg/cm$^2$) and its amount is such as to satisfy nearly all the steam requirements downstream of the I.D.R. loop. Data and results are in the following Table 1.

| Line (in figure 1) (°) | 2 | | 6 | | 7 | | 9 | |
|---|---|---|---|---|---|---|---|---|
| T (°C)<br>bar<br>P (kg/cm$^2$) | 130<br>196.1<br>(200) | | 205<br>196.1<br>(200) | | 172<br>196.1<br>(200) | | 200<br>196.1<br>(200) | |
| | kg/h | % | kg/h | % | kg/h | % | kg/h | % |
| $NH_3$ | — | — | 12466 | 36,1 | 3550 | 13,9 | 8916 | 47,9 |
| $CO_2$ | 9694 | 100 | 2698 | 7,8 | 2930 | 11,5 | 9462 | 50,8 |
| $H_2O$ | — | — | 6849 | 19,9 | 6600 | 25,8 | 249 | 1,3 |
| Urea | — | — | 12500 | 36,2 | 12500 | 48,8 | — | — |
| Total | 9694 | 100 | 34513 | 100 | 25580 | 100 | 18627 | 100 |

(°) The balance is approximated because of the exclusion of inerts and biuret; percentages are by weight.

Example 2 (Comparative)

The test of example 1 was repeated, while totally suppressing the injection of heating steam to the second ($CO_2$) stripper, operating therefore, in other words, in a full adiabatic way; data and results are reported in Table 2 hereinafter. The CBRU:CBRE ratio (in percentage) is=4646:2698×100=172.2%

| Line (in figure 1) | 2 | | 6 | | 7 | | 9 | |
|---|---|---|---|---|---|---|---|---|
| T (°C)<br>bar<br>P (kg/cm$^2$) | 130<br>196.1<br>(200) | | 205<br>196.1<br>(200) | | 165<br>196.1<br>(200) | | 200<br>196.1<br>(200) | |
| | kg/h | % | kg/h | % | kg/h | % | kg/h | % |
| $NH_3$ | — | — | 12466 | 36,1 | 4365 | 15,5 | 8101 | 50,5 |
| $CO_2$ | 9694 | 100 | 2698 | 7,8 | 4646 | 16,5 | 7746 | 48,3 |
| $H_2O$ | — | — | 6849 | 19,9 | 6650 | 23,6 | 199 | 1,2 |
| Urea | — | — | 12500 | 6,2 | 12500 | 44,4 | — | — |
| Total | 9694 | 100 | 34513 | 100 | 28161 | 100 | 16046 | 100 |

It is clear that the satisfactory removal of $NH_3$ is strictly bound to a considerable formation of carbamate, which (residual) carbamate must be then decomposed in the subsequent low-pressure sections of the plants, which implies a large and disadvantageous supply of heat. The downstream low-pressure steps would require therefore apparatuses having much greater dimensions and it is well known how difficult the recovery is at a sufficient thermal level, of the heat supplied for the low-pressure decomposition of the carbamate.

**Claims**

1. A process for the high-pressure removal of excess $NH_3$ at a pressure of from 78.4 to 245.2 bar (80 to 250 kg/cm$^2$) from an aqueous urea solution having a temperature of from 150 to 230°C and coming from a preceding stripping zone, isobaric with the synthesis of urea from $NH_3$ and $CO_2$, in which stripping is performed in the presence of $NH_3$, said urea solution also containing $CO_2$, as ammonium carbamate, in an amount equal to or lower than 25% by weight with respect to urea, and wherein said removal is performed in countercurrent to a $CO_2$ stream in a falling-film heat-exchanger containing a vertical tube bundle ($CO_2$ stripper) which is also isobaric with said urea synthesis and is heated by supplying steam, caracterized in that:

a) the global $NH_3$:$CO_2$ weight ratio in the solution fed to the $CO_2$ stripper is from 2 to 8; and

b) heat is supplied by means of steam substantially only to the uppermost portion of the tubes of the $CO_2$ stripper, the ratio between the surface of the lowermost portion and the surface of the uppermost portion of the tubes being at least 1:1 and the ratio between the residence time of the solution to be stripped in said lowermost portion and the residence time in said uppermost portion being at least 1:1, said residence time in the lowermost portion of the tubes being at least 3 seconds and said residence time in the uppermost portion being at most 3 seconds.

2. A process according to claim 1, wherein the surface of said uppermost portion of the tubes of the $CO_2$ stripper is from 1/5 to 1/2, preferably from 1/5 to 1/3, of the total surface of the tubes of said $CO_2$ stripper.

3. A process according to claim 1 or 2, wherein the pressure during the $CO_2$ stripping is from 127.4 to 215.8 bar, preferably 166.7 to 206 bar (130 to 220, preferably 170 to 210 kg/cm$^2$), and the inlet temperature of the urea solution fed to the $CO_2$ stripper is from 190 to 220, preferably 200 to 210°C.

4. A process according to any one of claims 1 to 3, wherein the outlet temperature of the final solution, leaving the bottom of the $CO_2$ stripper, is from 150 to 210, preferably 160 to 190, and more preferably 160 to 175°C provided said outlet temperature is lower than the inlet temperature at the top of the $CO_2$ stripper.

5. A process according to any one of claims 1 to 4, wherein the temperature of the $CO_2$ stream, injected as a stripping agent, is from 80 to 140°C.

6. A process according to any one of claims 1 to 5, wherein the ratio A between the exchanging surface of the $CO_2$ stripper ($S_{CO2}$) and the exchanging surface ($S_{NH3}$) of the preceding stripper is given by the expression:

$$A=S_{CO2}/S_{NH3}>1.$$

7. A process according to any one of claims 1 to 6, wherein the urea solution fed to the $CO_2$ stripper contains $CO_2$, as ammonium carbamate, in an amount equal to or lower than 22% by weight with respect to urea.

8. A process according to any one of claims 1 to 7, wherein the global $NH_3$:$CO_2$ weight ratio in the solution fed to the $CO_2$ stripper is from 3 to 6, preferably 4 to 5.5.

9. A process according to any one of claims 1 to 8, wherein the ratio between the surface of the lowermost portion of the tubes of the $CO_2$ stripper and the surface of the uppermost portion is at least 2:1, preferably 2:1 to 4:1.

10. A process according to any one of claims 1 to 9, wherein the ratio between the residence time of the solution to be stripped in said lowermost portion of the tubes of the $CO_2$ stripper and the residence time in said uppermost portion is at least 2:1, preferably 2:1 to 4:1, said residence time in the lowermost portion of the tubes being at least 3.5 s, preferably 3.5 to 5 s, and said residence time in the uppermost portion being at most 2.5 s, preferably 1.5 to 2.5 s.

**Patentansprüche**

1. Verfahren zur Hochdruckentfernung von überschüssigem $NH_3$ bei einem Druck von 78,4 bis 245,2 bar (80 bis 250 kg/cm$^2$) aus einer wässrigen Harnstofflösung mit einer Temperatur von 150 bis 230°C, die aus einer vorangehenden Strippzone kommt, welche mit der Harnstoffsynthese aus $NH_3$ und $CO_2$ isobar ist, bei der das Strippen in Gegenwart von $NH_3$ erfolgt, wobei die Harnstofflösung auch $CO_2$ als Ammoniumcarbonat in einer Menge gleich oder niedriger als 25 Gew.-%, bezogen auf Harnstoff, enthält, und wobei das Abtrennen im Gegenstrom zu einem $CO_2$-Strom in einem Fallfilm-Wärmeaustauscher durchgeführt wird, der ein vertikales Rohrbündel enthält ($CO_2$-Stripper), welcher ebenfalls isobar mit der Harnstoffsynthese ist und durch Zufuhr von Dampf beheizt wird, dadurch gekennzeichnet, daß:

a) das globale $NH_3$:$CO_2$-Gewichtsverhältnis in der dem $CO_2$-Stripper zugeführten Lösung 2 bis 8 beträgt; und

b) Wärme mittels Dampf im wesentlichen nur dem obersten Bereich der Rohre des $CO_2$-Strippers zugeführt wird, wobei das Verhältnis der Oberfläche des untersten Bereichs zu der Oberfläche des obersten Bereichs der Rohre mindestens 1:1 beträgt und das Verhältnis zwischen der Verweilzeit der in dem untersten Bereich zu strippenden Lösung und der Verweilzeit in dem obersten Bereich mindestens 1:1 beträgt, wobei die Verweilzeit in dem untersten Bereich der Rohre mindestens 3 Sekunden und die Verweilzeit in dem obersten Bereich höchstens 3 Sekunden beträgt.

2. Verfahren nach Anspruch 1, worin die Oberfläche des obersten Bereichs der Rohre des $CO_2$-Strippers 1/5 bis 1/2, vorzugsweise 1/5 bis 1/3, der Gesamtoberfläche der Rohre des $CO_2$-Strippers ausmacht.

3. Verfahren nach Anspruch 1 oder 2, worin der Druck während des $CO_2$-Strippens 127,4 bis 215,8 bar, vorzugsweise 166,7 bis 206 bar (130 bis 220, vorzugsweise 170 bis 210 $kg/cm^2$), und die Einlaßtemperatur der dem $CO_2$-Stripper zugeführten Harnstofflösung 190 bis 220, vorzugsweise 200 bis 210°C, betragen.

4. Verfahren nach irgendeinem der Ansprüche 1 bis 3, worin die Auslaßtemperatur der Endlösung, die den Boden des $CO_2$-Strippers verläßt, 150 bis 210, vorzugsweise 160 bis 190 und besonders bevorzugt 160 bis 175°C beträgt, vorausgesetzt, daß die Auslaßtemperatur niedriger ist als die Einlaßtemperatur am Kopf des $CO_2$-Strippers.

5. Verfahren nach irgendeinem der Ansprüche 1 bis 4, worin die Temperatur des $CO_2$-Stroms, der als Strippmittel eingeleitet wird, 80 bis 140°C beträgt.

6. Verfahren nach irgendeinem der Ansprüche 1 bis 5, worin das Verhältnis A zwischen der Austauschoberfläche des $CO_2$-Strippers ($S_{CO2}$) und der Austauschoberfläche ($S_{NH3}$) des vorangehenden Strippers durch folgenden Ausdruck wiedergegeben wird:

$$A=S_{CO2}/S_{NH3}>1.$$

7. Verfahren nach irgendeinem der Ansprüche 1 bis 6, worin die dem $CO_2$-Stripper zugeführte Harnstofflösung $CO_2$ als Ammoniumcarbonat in eine Menge gleich oder niedriger als 22 Gew.-%, bezogen auf Harnstoff, enthält.

8. Verfahren nach irgendeinem der Ansprüche 1 bis 7, worin das globale $NH_3$:$CO_2$-Gewichtsverhältnis in der dem $CO_2$-Stripper zugeführten Lösung 3 bis 6, vorzugsweise 4 bis 5,5, beträgt.

9. Verfahren nach irgendeinem der Ansprüche 1 bis 8, worin das Verhältnis zwischen der Oberfläche des untersten Bereiches der Rohre des $CO_2$-Strippers und der Oberfläche des obersten Bereiches mindestens 2:1, vorzugsweise 2:1 bis 4:1, beträgt.

10. Verfahren nach irgendeinem der Ansprüche 1 bis 9, worin das Verhältnis zwischen der Verweilzeit der in dem untersten Bereich der Rohre des $CO_2$-Strippers zu strippenden Lösung und der Verweilzeit in dem obersten Bereich mindestens 2:1, vorzugsweise 2:1 bis 4:1, die Verweilzeit in dem untersten Bereich der Rohre mindestens 3,5 s, vorzugsweise 3,5 bis 5 s, und die Verweilzeit in dem obersten Bereich höchstens 2,5 s, vorzugsweise 1,5 bis 2,5 s, betragen.

**Revendications**

1. Un procédé pour l'élimination sous pression élevée de l'excès de $NH_3$ à une pression de 78,4 à 245,2 bars (80 à 250 $kg/cm^2$) d'une solution aqueuse d'urée présentant une température de 150 à 230°C et provenant d'une zone d'extraction précédente, opérant à la même pression que la pression de la synthèse d'urée à partir de $NH_3$ et de $CO_2$, mise en oeuvre en présence de $NH_3$, cette solution d'urée contenant également du $CO_2$, sous la forme de carbamate d'ammonium, en quantité égale ou inférieure à 25% en poids par rapport à l'urée et dans lequel ladite élimination est mise en oeuvre à contrecourant d'un courant de $CO_2$, dans un extracteur thermique, à film ruisselant, contenant un faisceau de tubes verticaux (extraction par $CO_2$) qui opère également sous la même pression que celle de la synthèse de l'urée et qui est chauffé par apport externe de vapeur, caractérisé en ce que:

a) le rapport en poids global de $NH_3/CO_2$ dans la solution introduite dans l'appareil d'extraction par $CO_2$ est compris entre 2 et 8; et

b) la chaleur est amenée presque uniquement par de la vapeur à la partie supérieure des tubes de l'appareil d'extraction par $CO_2$, le rapport entre la surface de la partie inférieure et la surface de la partie supérieure des tubes étant d'au moins 1/1 et le rapport entre la durée de séjour de la solution à extraire dans la partie inférieure et la durée de séjour dans la partie supérieure étant d'au moins 1/1, cette durée de séjour dans la partie inférieure des tubes étant d'au moins 3 secondes et cette durée de séjour dans la partie supérieure étant d'au moins 3 secondes.

2. Un procédé selon la revendication 1, dans lequel la surface de la partie supérieure des tubes de l'appareil d'extraction par $CO_2$ est comprise entre 1/5 et 1/2, de préférence 1/5 et 1/3, de la surface totale des tubes dudit appareil d'extraction par $CO_2$.

3. Un procédé selon la revendication 1 ou 2, dans lequel la pression durant le traitement d'extraction par $CO_2$ est comprise entre 127,4 et 215,8 bars, de préférence 166,7 et 206 bars (130 à 220, de préférence 170

à 210 kg/cm$^2$) et la température d'entrée de la solution d'urée introduite dans l'appareil d'extraction par $CO_2$ est comprise entre 190 et 220, de préférence 200 et 210°C.

4. Un procédé selon l'une quelconque des revendications 1 à 3, dans lequel la température de sortie de la solution finale, quittant la cuve de l'appareil d'extraction par $CO_2$ est comprise entre 150 et 210, de préférence 160 et 190, et plus particulièrement 160 et 175, étant entendu que ladite température à la sortie est inférieure à la température d'entrée en tête de l'appareil d'extraction par $CO_2$.

5. Un procédé selon l'une quelconque des revendications 1 à 4, dans lequel la température du courant de $CO_2$ injecté en tant qu'agent d'extraction est compris entre 80 et 140°C.

6. Un procédé selon l'une quelconque des revendications 1 à 5, dans lequel le rapport A entre la surface d'échange de l'appareil d'extraction par $CO_2$ ($S_{CO2}$) et la surface d'échange de l'appareil d'extraction précédente ($S_{NH3}$) est exprimé par la formule:

$$A=S_{CO2}/S_{NH3}>1$$

7. Un procédé selon l'une quelconque des revendications 1 à 6, dans lequel la solution d'urée introduite dans l'appareil d'extraction par $CO_2$ contient du $CO_2$ sous la forme de carbamate d'ammonium, en une quantité égale ou inférieure à 22% en poids par rapport à l'urée.

8. Un procédé selon l'une quelconque des revendications 1 à 7, dans lequel le rapport pondéral $NH_3/CO_2$ total dans la solution introduite dans l'appareil d'extraction par $CO_2$ est compris entre 3 et 6, de préférence 4 et 5,5.

9. Un procédé selon l'une quelconque des revendications 1 à 8, dans lequel le rapport entre la surface de la partie inférieure des tubes de l'appareil d'extraction par $CO_2$ et la surface de la partie supérieure est d'au moins 2/1, de préférence 2/1 et 4/1.

10. Un procédé selon l'une quelconque des revendications 1 à 9, dans lequel le rapport entre la durée de séjour de la solution à entraîner dans ladite partie inférieure des tubes de l'appareil d'extraction par $CO_2$ et la durée de séjour dans la partie inférieure est d'au moins 2/1, de préférence comprise entre 2/1 et 4/1, ladite durée de séjour à la partie inférieure des tubes étant d'au moins 3,5 secondes, de préférence 3,5 à 5 secondes, et ladite durée de séjour dans la partie supérieure étant d'au moins 2,5 secondes, de préférence 1,5 à 2,5 secondes.

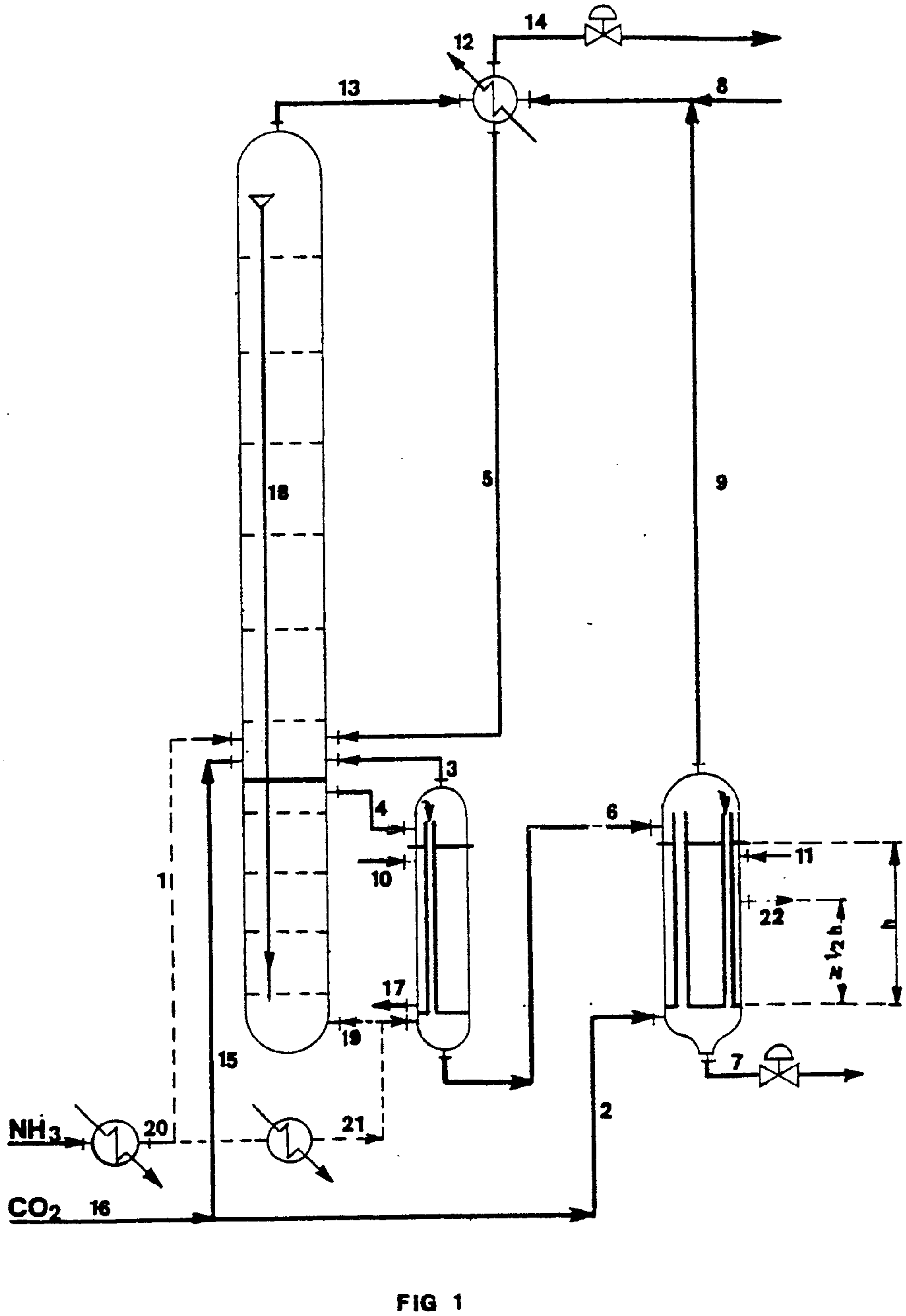


FIG 1